# EUROPEAN PATENT APPLICATION

(11) **EP 2 912 987 A1**
(43) Date of publication of application: **02.09.2015**
(21) Application number: 13849891.0
(22) Date of filing: 23.10.2013
(51) Int. Cl.: A61B 1/00, G02B 23/24

(54) **INSERTION SYSTEM, INSERTION SUPPORT DEVICE, INSERTION SUPPORT METHOD AND PROGRAM**

(30) Priority: 25.10.2012 JP 2012235634
(71) Applicant: Olympus Corporation, Shibuya-ku Tokyo 151-0072 (JP)
(72) Inventor: FUJITA, Hiromasa, Tokyo 151-0072 (JP); TOJO, Ryo, Tokyo 151-0072 (JP)
(74) Representative: Winter, Brandl, Fürniss, Hübner, Röss, Kaiser, Polte - Partnerschaft mbB
(86) International application number: PCT/JP2013/078738
(87) International publication number: WO 2014/065336

(57) **Abstract**

An inserting state acquiring section (61) acquires inserting state information of an inserting section that is to be inserted into an insertion subject, an insertion subject shape acquiring section (62) acquires shape information of the insertion subject, and a positional relation calculating section (63) is input the inserting state information and the insertion subject shape information and calculates a positional relation of the inserting section to the insertion subject. When an output section (64) outputs the calculation result of the positional relation calculating section (63), a control section (66) controls an output state of the calculation result in the output section (64).

## Description

### Technical Field

The present invention relates to an insertion system in which an inserting section of an inserting tool such as an endoscope or a catheter is inserted into an insertion subject to perform an observation or an operation, an insertion supporting device which supports the insertion of the inserting section into the insertion subject in such an insertion system, an insertion supporting method, and a program which allows a computer to execute a procedure of the insertion supporting device.

### Background Art

As a supporting device in a case where an inserting section is inserted into an insertion subject for observation, for example, there is disclosed, in U.S. Patent No. 6,846,286, a constitution to display a shape of an endoscope inserting section in a display section when the endoscope inserting section is inserted into a human body.

As to this constitution, in an endoscope device, flexible bend detecting optical fibers having bend detecting portions in which a quantity of light to be transmitted changes in accordance with a size of an angle of a bend are attached to a flexible band-like member in a state where the fibers are arranged in parallel, and the band-like member is inserted into and disposed in the endoscope inserting section along a substantially total length of the endoscope inserting section. Additionally, a bending state of the band-like member in a portion where each bend detecting portion is positioned is detected from the light transmission quantity of each bend detecting optical fiber, to display the bending state as the bending state of the endoscope inserting section in a monitor screen.

In general, there are only a few regions that become marks in an insertion subject, and hence when it is not easily judged only from an acquired image which region of the insertion subject is being observed, it is also not easily judged whether or not all required regions could be imaged (observed).

In a technology disclosed in U.S. Patent No. 6,846,286 mentioned above, it is possible to display a shape of an inserting section in the insertion subject which cannot be seen from the outside of the insertion subject when the inserting section is inserted into the insertion subject. However, there has not been suggested a method of detecting and displaying which region of the insertion subject is being imaged (observed).

Additionally, also in a tubular inserting tool such as a catheter that does not have an image acquisition section but is inserted into an insertion subject to perform a predetermined operation, there are desired detection of which region of the insertion subject is being operated and a display method of the detection.

### Summary of Invention

The present invention has been developed in view of the above respects, and an object thereof is to provide an insertion system, an insertion supporting method and a program that can supply, to an operator, information to judge which position of an insertion subject is being acquired image or which position is being operated, an insertion supporting device.

According to a first aspect of the invention, there is provided an insertion system characterized by comprising an inserting section that is to be inserted into an insertion subject, an inserting state acquiring section that acquires inserting state information of the inserting section, an insertion subject shape acquiring section that acquires shape information of the insertion subject, a positional relation calculating section that is input the inserting state information and the insertion subject shape information and calculates a positional relation of the inserting section to the insertion subject, an output section that outputs the calculation result of the positional relation calculating section, and a control section that controls an output state of the calculation result in the output section.

According to a second aspect of the invention, there is provided an insertion supporting device which supports insertion of an inserting section of an inserting tool into an insertion subject, the insertion supporting device characterized by comprising an inserting state acquiring section that acquires inserting state information of the inserting section, an insertion subject shape acquiring section that acquires shape information of the insertion subject, a positional relation calculating section that is input the inserting state information and the insertion subject shape information and calculates a positional relation of the inserting section to the insertion subject, an output section that outputs the calculation result of the positional relation calculating section as display information, and a control section that controls an output state of the calculation result in the output section.

According to a third aspect of the invention, there is provided an insertion supporting method for use in an insertion supporting device which supports insertion of an inserting section of an inserting tool into an insertion subject, the method characterized by comprising an inserting state acquiring step of acquiring inserting state information of the inserting section, an insertion subject shape acquiring step of acquiring shape information of the insertion subject, a positional relation calculating step of being input the inserting state information and the insertion subject shape information, and calculating a positional relation of the inserting section to the insertion subject, an output step of outputting the calculation result of the positional relation calculating step as display information, and a control step that controlling an output state of the calculation result in the output step.

According to a forth aspect of the invention, there is provided a program which allows a computer to execute an insertion state acquiring procedure of acquiring inserting state information of the inserting section in an insertion supporting device which supports insertion of the inserting section of an inserting tool into an insertion subject, an insertion subject shape acquiring procedure of acquiring shape information of the insertion subject, a positional relation calculating procedure of being input the inserting state information and the insertion subject shape information, and calculating a positional relation of the inserting section to the insertion subject, an output procedure of outputting the calculation result of the positional relation calculating procedure as display information, and a control procedure of controlling an output state of the calculation result in the output procedure.

According to the present invention, there can be provided an insertion system, an insertion supporting device, an insertion supporting method and a program that can supply information to judge which position of an insertion subject is being acquired image or operated and therefore enables an operator to easily judge which region of the insertion subject is being acquired image or operated and whether all required regions could be acquired images or operated, so that it is possible to prevent oversight of observation or operation regions.

Additionally, according to the present invention, when a positional relation of an inserting section to an insertion subject which is calculated by a positional relation calculating section is output by an output section, a control section sets at least a start point of the output, and in this case, the positional relation is not always output but is output only when necessary, so that useless information is not supplied but the output can be easy to understand (easy to see, hard to be mistaken, or can quickly be judged).

### Brief Description of Drawings

FIG. 1A is a view showing a schematic constitution of an insertion system to which an insertion supporting device according to a first embodiment of the present invention is applied;
FIG. 1B is a block diagram of the insertion supporting device according to the first embodiment;
FIG. 1C is a view for explaining a supply example of information by an output section of the insertion supporting device according to the first embodiment;
FIG. 2A is a view showing a schematic constitution of a soft endoscope device as an inserting tool in the insertion system according to the first embodiment;
FIG. 2B is a perspective view of a distal end of an inserting section;
FIG. 3A is a view for explaining a constitution of an insertion and rotation detecting section;
FIG. 3B is a view for explaining an operation principle of the insertion and rotation detecting section;
FIG. 4 is a view showing an inserting state of the inserting section into an insertion subject;
FIG. 5A is a view showing a case where a bending portion is bent in an upward direction of the paper surface to explain a principle of a fiber shape sensor;
FIG. 5B is a view showing a case where the bending portion is not bent to explain the principle of the fiber shape sensor:
FIG. 5C is a view showing a case where the bending portion is bent in a downward direction of the paper surface to explain the principle of the fiber shape sensor;
FIG. 6 is a view showing an attaching structure of the fiber shape sensor to the inserting section;
FIG. 7 is a block diagram of an insertion supporting device according to a third embodiment;
FIG. 8A is a view for explaining an example of an inlet/outlet of the insertion subject;
FIG. 8B is a view for explaining another example of the inlet/outlet of the insertion subject;
FIG. 8C is a view for explaining still another example of the inlet/outlet of the insertion subject;
FIG. 8D is a view for explaining a further example of the inlet/outlet of the insertion subject;
FIG. 9A is a view for explaining which position of the insertion subject is displayed by a first position display;
FIG. 9B is a view for explaining which position of the insertion subject is displayed by a second position display;
FIG. 10 is a diagram showing a timing chart for explaining an output timing of the output section by control of a control section;
FIG. 11 is a view for explaining another example of an information supply configuration;
FIG. 12A is a view for explaining a display example in a case where the inserting section is inserted into a branched insertion subject, as a still further example of the supply configuration of the information;
FIG. 12B is a view for explaining another display example;
FIG. 12C is a view for explaining still another display example;
FIG. 13A is a view showing a state before rotation to explain a change of an acquired image due to the rotation of the inserting section;
FIG. 13B is a view showing a state after the rotation to explain the change of the acquired image due to the rotation of the inserting section;
FIG. 14A is a view for explaining another example of the information supply configuration;
FIG. 14B is a view for explaining still further example of the information supply configuration;
FIG. 14C is a view for explaining another example of the information supply configuration;
FIG. 14D is a view for explaining still further example of the information supply configuration;
FIG. 14E is a view for explaining another example of the information supply configuration;
FIG. 14F is a view for explaining still further example of the information supply configuration;
FIG. 15 is a view for explaining still further example of the information supply configuration;
FIG. 16A is a block constitutional view of an insertion system according to a second embodiment of the present invention;
FIG. 16B is a view for explaining an example of supply of information by a display device of the insertion system according to the second embodiment and an output section of an insertion supporting device according to the second embodiment of the present invention;
FIG. 17 is a view showing a schematic constitution of a hard endoscope device as an inserting tool in an insertion system according to a third embodiment of the present invention; and
FIG. 18 is diagram showing a schematic constitution of a catheter as an inserting tool in an insertion system according to a fourth embodiment of the present invention.

### Description of Embodiments

Hereinafter, modes for carrying out the present invention will be described with reference to the drawings.

### [First Embodiment]

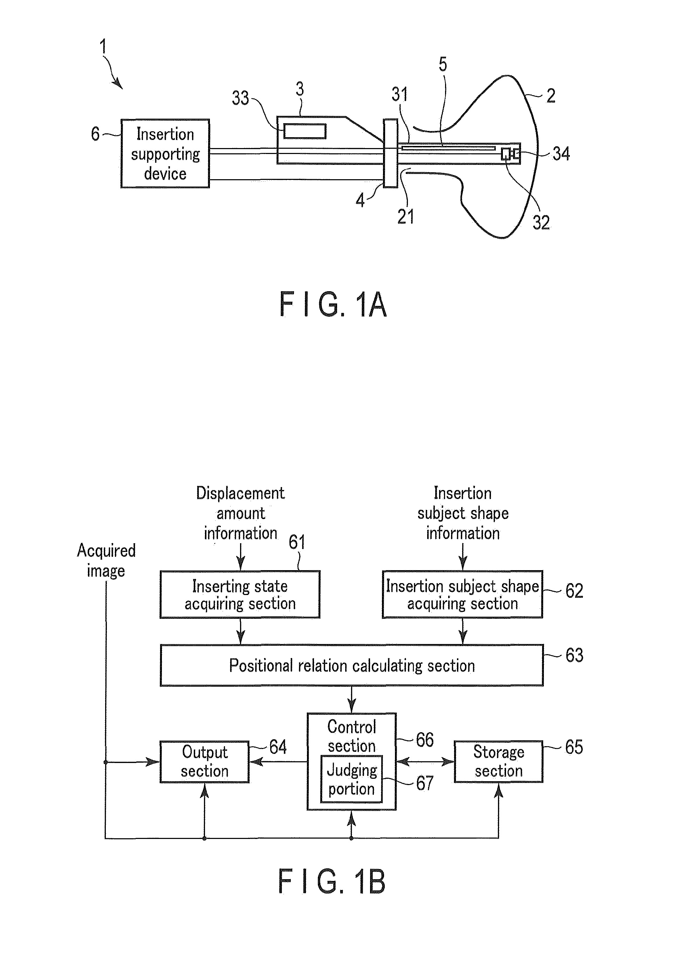

As shown in FIG. 1A, an insertion system 1 according to a first embodiment of the present invention is constituted of an inserting tool 3 including an inserting section 31 to be inserted into an insertion subject 2 and an image acquisition section 32 that acquires image of the insertion subject 2, an insertion and rotation detecting section 4 and a fiber shape sensor 5 as detecting sections that detect displacement amount information of the inserting section 31, and an insertion supporting device 6 concerned with the first embodiment of the present invention which acquires inserting state information of the inserting section 31 from the displacement amount information from the insertion and rotation detecting section 4 and the fiber shape sensor 5, and calculates a positional relation of the inserting section 31 in relation to the insertion subject 2 on the basis of the inserting state information and shape information of the insertion subject 2 to display and output the calculation result.

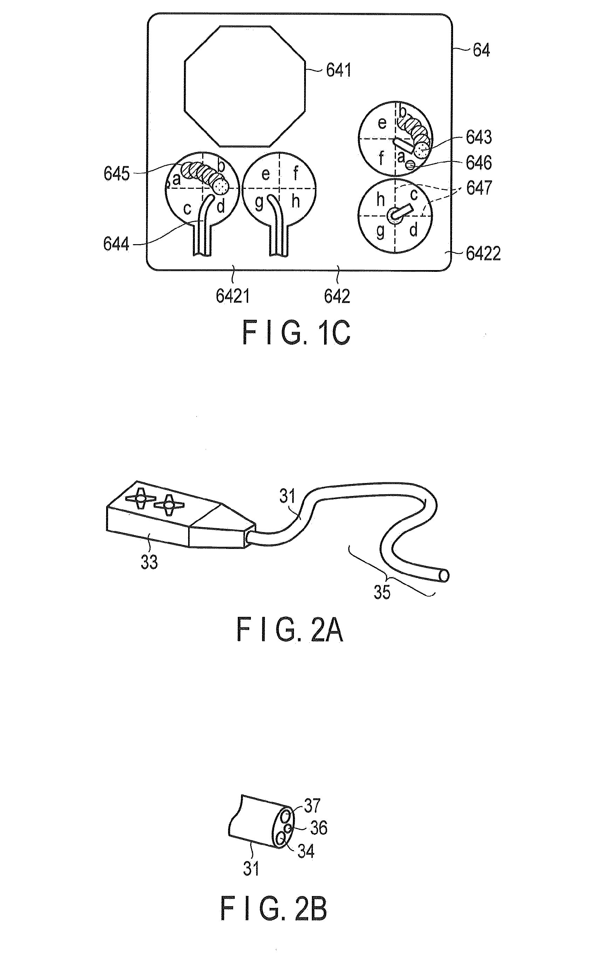

Hereinafter, it will be described in the present embodiment that the inserting tool 3 is such a soft endoscope device as shown in FIG. 2A. This soft endoscope device includes the inserting section 31, and an operating section 33 constituted integrally with the inserting section 31. The inserting section 31 is a flexible tubular member, and is insertable from an insertion port 21 of the insertion subject 2 into the insertion subject 2. It is to be noted that the insertion subject 2 is filled with a predetermined material such as air, physiological saline or a chemical solution. In an end portion of the inserting section 31 in an inserting direction (hereinafter referred to as an inserting section distal end), as shown in FIG. 2B, an image acquisition opening 34 is disposed, and in the vicinity of the inserting section distal end in the inserting section 31, as shown in FIG. 1A, the image acquisition section 32 is included. Light entering into the image acquisition opening 34 is received by the image acquisition section 32 that performs image acquisition. An image acquired by the image acquisition section 32 is displayed and output in the insertion supporting device 6 concerned with the present first embodiment.

It is to be noted that needless to say, the image acquisition section 32 may not be disposed in the vicinity of the inserting section distal end of the inserting section 31 but may be disposed in the operating section 33 and connected to the image acquisition opening 34 by a light guide or the like to guide the light entering into the image acquisition opening 34 to the image acquisition section 32 that performs the image acquisition.

In addition, the inserting section 31 includes a bending portion 35 in the vicinity of the inserting section distal end, and the bending portion 35 is coupled to an operation lever disposed in the operating section 33 by a wire, though not shown in the drawing. In consequence, the operation lever is moved to pull the wire, thereby enabling a bending operation of the bending portion 35.

In addition, although not especially shown in the drawing, the inserting section 31 has an illuminating optical fiber therein, and light from an unshown illuminating light source disposed in the operating section 33 is guided to exit as illumination light for the image acquisition from a light supplying portion 36 at the inserting section distal end. Further, at the inserting section distal end, a treating opening 37 is disposed, and a treatment tool inserted from the operating section 33 into the inserting section 31 can extend from the treating opening 37 to the outside of the inserting section 31.

Hereinafter, a constitution of each section will be described in detail.

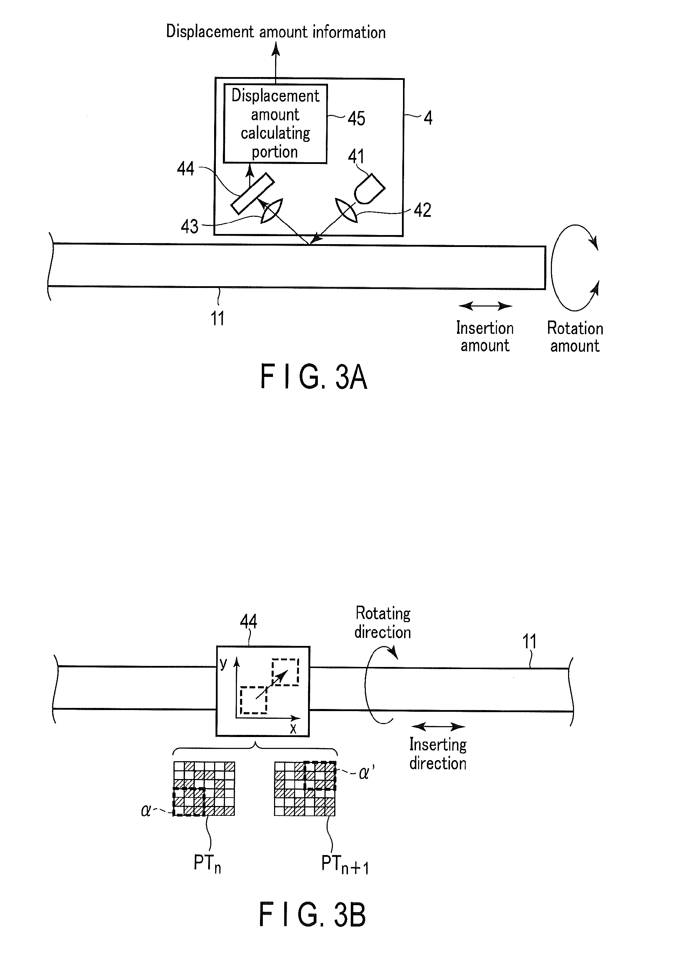

In addition, the insertion and rotation detecting section 4 is disposed in the vicinity of the insertion port 21 of the insertion subject 2, and detects an insertion amount and a rotation amount of the inserting section 31 to output the amounts as one piece of the displacement amount information of the inserting section 31 to the insertion supporting device 6. Specifically, as shown in FIG. 3A, the insertion and rotation detecting section 4 is constituted of a light source 41, a projection lens 42, a light receiving lens 43, an optical pattern detecting portion 44, and a displacement amount calculating portion 45.

The inserting section 31 is irradiated with the light emitted from the light source 41 through the projection lens 42, and the light reflected by the inserting section 31 is received through the light receiving lens 43 by the optical pattern detecting portion 44. The optical pattern detecting portion 44 detects images of a plane of the inserting section 31 which is an optical pattern continuously at detection times t₀, t₁, t₂, ..., tₙ, ....

As shown in FIG. 3B, the displacement amount calculating portion 45 compares a displacement in image data of any selected reference pattern α that is present in the image (an optical pattern PTₙ) of the image data acquired at any time tₙ by the optical pattern detecting portion 44 with a displacement in image data of an optical pattern α' that is present in a part of an image (an optical pattern PTₙ₊₁) of the image data acquired at any time tₙ₊₁ after the elapse of time from the above time tₙ and that matches the above reference pattern α, and the displacement amount calculating portion calculates a displacement amount in each image in an x-axis direction and a y-axis direction. Here, as shown in FIG. 3B, the optical pattern detecting portion 44 is positioned so that an x-axis of the optical pattern detecting portion 44 matches an axial direction of the inserting section 31. Therefore, a displacement amount Δx_{f} in the x-axis direction which is calculated by the displacement amount calculating portion 45 is proportional to the insertion amount of the inserting section 31, and a displacement amount Δy_{f} in the y-axis direction is proportional to the rotation amount of the inserting section 31. The displacement amounts, (the insertion amount and the rotation amount) in the images which are calculated by the displacement amount calculating portion 45 are output as the displacement amount information to the insertion supporting device 6. It is to be noted that an increase/decrease direction of each displacement amount indicates directions of insertion and rotation of the inserting section 31, and hence the displacement amount information also includes information of the inserting direction and the rotating direction.

It is to be noted that the acquisition of the displacement amount information is not limited to the above method, and the information may be obtained on the basis of image data.

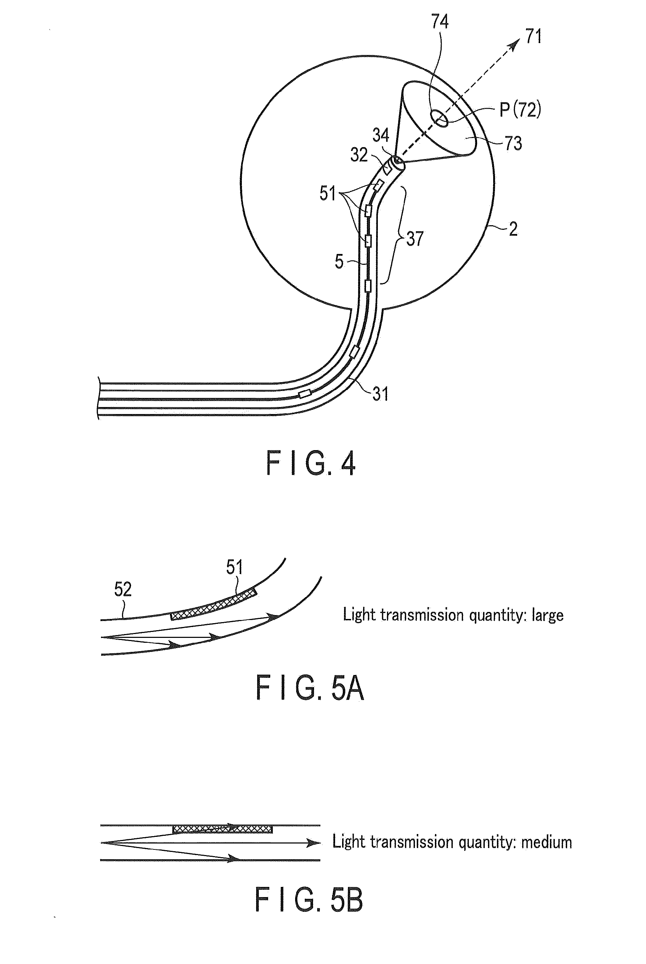

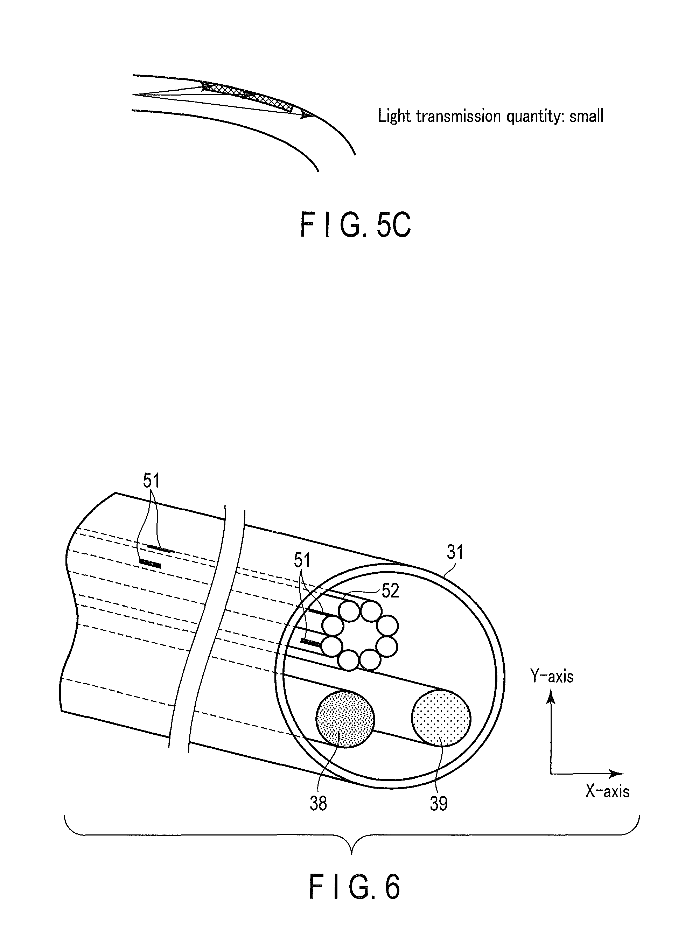

Additionally, as shown in FIG. 4, the fiber shape sensor 5 is disposed in the inserting section 31. The fiber shape sensor 5 is constituted of optical fibers, and one of the optical fibers has a bend detecting portion 51 of one portion. In the bend detecting portion 51, a clad of the optical fiber is removed to expose a core thereof, and a light absorbing material is applied to constitute the bend detecting portion. In the bend detecting portion 51, as shown in FIG. 5A to FIG. 5C, a quantity of light to be absorbed by the bend detecting portion 51 changes in accordance with a bend of the bending portion 35, and hence a quantity of the light to be guided in an optical fiber 52 changes, i.e., a light transmission quantity changes. It is to be noted that in the present embodiment, the fiber shape sensor 5 is constituted of the optical fibers, but the present invention is not limited to this example, and the sensor may be a single optical fiber.

In the fiber shape sensor 5 of this constitution, for the purpose of detecting the bend in an X-axis direction and the bend in a Y-axis direction shown in FIG. 6, two optical fibers 52 are disposed so that the two bend detecting portions 51 directed in the X-axis direction and the Y-axis direction, respectively, form a pair, to detect a bend amount of one region. Furthermore, the optical fibers 52 are disposed so that the pair of bend detecting portions 51 are arranged in a longitudinal direction (an inserting direction) of the inserting section 31. Furthermore, light from an unshown light source is guided by each of the optical fibers 52, and the light transmission quantity that changes with the bend amount of each of the optical fibers 52 is detected by an unshown light receiving section. The thus detected light transmission quantities are output as one piece of displacement amount information of the inserting section 31 to the insertion supporting device 6.

It is to be noted that the bend detecting portions 51 are preferably disposed not only in the bending portion 35 of the inserting section 31 but also on an operating section side from the bending portion, so that it is possible to also detect a bending state of a portion other than the bending portion 35 of the inserting section 31, which freely bends in accordance with an internal structure of the insertion subject 2 due to flexibility of the inserting section 31.

It is to be noted that as shown in FIG. 6, an illuminating optical fiber 38 and a wiring line 39 for the image acquisition section are also disposed in the inserting section 31. The light from the unshown illuminating light source disposed in the operating section 33 is guided by the illuminating optical fiber 38, and emitted as illuminating light from the inserting section distal end, so that the image acquisition section 32 can acquire image of the inside of the insertion subject 2 that is a dark part.

Additionally, as shown in FIG. 1B, the insertion supporting device 6 concerned with the present embodiment is constituted of an inserting state acquiring section 61, an insertion subject shape acquiring section 62, a positional relation calculating section 63, an output section 64, and a storage section 65, and control section 66.

The inserting state acquiring section 61 acquires inserting state information of at least a part of the inserting section 31 inserted into the insertion subject 2, e.g., a position and a direction of a certain point of the inserting section 31 on the basis of the displacement amount information output from the displacement amount calculating portion 45 of the insertion and rotation detecting section 4. In addition, on the basis of the light transmission quantity that changes with the bend amount of each of the optical fibers 52 as the displacement amount information detected by the fiber shape sensor 5, the inserting state acquiring section further acquires a position and a direction of each of the bend detecting portions 51 of the inserting section 31 as the inserting state information into the insertion subject 2.

The insertion subject shape acquiring section 62 acquires the shape information of the insertion subject 2 (the insertion subject shape information). This insertion subject shape information is constituted on the basis of data from the outside or inside of the insertion subject 2 before the inserting section 31 is inserted into the insertion subject 2.

That is, the insertion subject shape information based on the data from the outside is constituted by utilizing an apparatus that can detect the information by use of the light transmitted through the insertion subject 2, for example, a CT diagnosis apparatus, an ultrasonic diagnosis apparatus or an X-ray apparatus.

In addition, the insertion subject shape information based on the data from the inside is constituted by utilizing locus data obtained when the inserting section 31 is moved in a space of the insertion subject 2 or by connecting position information obtained when the inserting section distal end comes in contact with the insertion subject 2. When the position information obtained during the contact between the inserting section distal end and the insertion subject 2 is utilized, a size of the space can be detected, and the insertion subject shape information can more exactly be acquired. Furthermore, when the insertion subject 2 is a human organ, the information may be constituted by presuming a physical constitution, and when the insertion subject 2 is a structure, the information may be constituted by inputting the shape through a drawing.

It is to be noted that when the insertion subject shape information is acquired by the insertion subject shape acquiring section 62, the insertion subject shape information may directly be acquired from an apparatus such as the CT diagnosis apparatus by connecting the apparatus that constitutes the insertion subject shape information, or the insertion subject shape information may be acquired by storing the insertion subject shape information output from the apparatus once in a storage medium and reading the stored insertion subject shape information or by downloading the insertion subject shape information via a network. Furthermore, the insertion subject shape acquiring section 62 is not limited to that interface or data reader and the acquiring section itself may be the apparatus that constitutes the insertion subject shape information.

The positional relation calculating section 63 calculates a positional relation of the inserting section 31 in relation to the insertion subject 2, i.e., a specific position of the insertion subject 2 to which the whole inserting section 31 or the distal end of the inserting section is directed, on the basis of the inserting state information acquired by the inserting state acquiring section 61 and the insertion subject shape information acquired by the insertion subject shape acquiring section 62. Specifically, the positional relation calculating section 63 first calculates shape information of the inserting section 31 on the basis of the position and direction of each of the bend detecting portions 51 of the inserting section 31 as the inserting state information acquired by the inserting state acquiring section 61. Furthermore, on the basis of this obtained shape information of the inserting section 31, a position and a direction of, for example, a certain point of the inserting section 31 as the above inserting state information acquired by the inserting state acquiring section 61, and the above insertion subject shape information acquired by the insertion subject shape acquiring section 62, the positional relation calculating section calculates the positional relation of the inserting section 31 in relation to the insertion subject 2, i.e., a position of the inserting section distal end and a direction (an axial direction) in which the distal end is directed. Furthermore, an intersection between the direction in which the inserting section distal end is directed and the insertion subject 2 is calculated on the basis of the calculation results and the insertion subject shape information. That is, as shown in FIG. 4, the positional relation calculating section 63 obtains, as an image acquisition position P, an intersection 72 between a straight line including a direction in which the inserting section distal end is directed (an image acquisition direction 71) and the shape of the insertion subject 2, i.e., the center of a viewing field (an image acquisition region 73).

In general, a region of interest in an observation object is at the center of the viewing field, and hence the center of the viewing field is often more important than a periphery thereof. It is to be noted that here the description has been given as to the example where the intersection is obtained as the image acquisition position P, but the viewing field (the image acquisition region 73) that is a region of the insertion subject 2 imaged by the image acquisition section 32 may be calculated as the image acquisition position P from a distance between the position of the inserting section distal end and an image acquisition plane of the insertion subject 2 on the basis of the insertion subject shape information. In this case, when parameters such as a refractive index of the predetermined material interposed between the inserting section 31 and the insertion subject 2 and view angle information of the image acquisition section 32 (a focal length of a lens or the like) is used, the image acquisition region 73 can more accurately be obtained. The image acquisition region 73 is obtained as the image acquisition position P in this manner, so that a region imaged by the image acquisition section 32 can be grasped. In addition, a partial region 74 or a point in the viewing field (the image acquisition region 73) may be calculated as the image acquisition position P. For example, when the image acquisition region 73 cannot exactly be detected, a small region is calculated in consideration of an error, so that a region that is not imaged can be prevented from being wrongly detected as the imaged region. That is, an omission of observation can be prevented.

The positional relation calculating section 63 outputs, as the calculation results to the control section 66, the obtained shape information of the inserting section 31, the obtained positional relation of the inserting section 31 in relation to the insertion subject 2, i.e., the position of the inserting section distal end and the direction (the axial direction) in which the distal end is directed, the above insertion subject shape information acquired by the insertion subject shape acquiring section 62 and image acquisition position information indicating the obtained image acquisition position P (e.g., the intersection 72).

The output section 64 performs the display output in a configuration where an operator can judge a specific position of the insertion subject 2 to which the inserting section distal end is directed, from the above calculation result of the positional relation calculating section 63 under the control of the control section 66. In addition, the output section 64 also functions as a display section that can display the above image acquired from the image acquisition section 32.

The storage section 65 stores the above calculation result of the positional relation calculating section 63 and also stores the image acquired from the image acquisition section 32 under the control of the control section 66.

The control section 66 controls an output state of the above display output of the above calculation result of the positional relation calculating section 63 in the output section 64 and the storage of the calculation result in the storage section 65. That is, the above calculation results from the positional relation calculating section 63 are successively output to the control section 66, but the control section 66 does not allow the output section 64 to display and output all the output calculation results. Concerning the image acquisition position information indicating the image acquisition position P (e.g., the intersection 72) which is at least part of the calculation results, the control section controls presence/absence of the display output, i.e., a display output start point and a display output end point, and controls a type of display output of the calculation result, i.e., a method of display. Similarly, also concerning the storage into the storage section 65, the control section executes the control so that all the above calculation results output from the positional relation calculating section 63 are not stored but at least part of the calculation results is stored. In addition, not only the storage of the calculation result of the positional relation calculating section 63 but also the storage of the acquired image from the image acquisition section 32 can similarly be controlled.

For example, the control section 66 can include a judging portion 67 that judges whether the inserting section distal end has reached a predetermined position. Here, the predetermined position indicates a region of the insertion subject 2 which is required to be observed or treated. The judging portion 67 performs this judgment on the basis of part of the above calculation results output from the positional relation calculating section 63, i.e., the shape information of the inserting section 31, the position of the inserting section distal end and the direction (the axial direction) in which the distal end is directed, and the insertion subject shape information, or the judging portion performs the judgment from the above image acquired from the image acquisition section 32 by pattern matching or the like. Furthermore, the control section 66 controls the output section 64 and the storage section 65 in accordance with this judgment result of the judging portion 67.

In addition, the control section 66 also has a function of sending the information (the above calculation result of the positional relation calculating section 63, the acquired image, etc.) stored in the storage section 65 to the output section 64 to display the information.

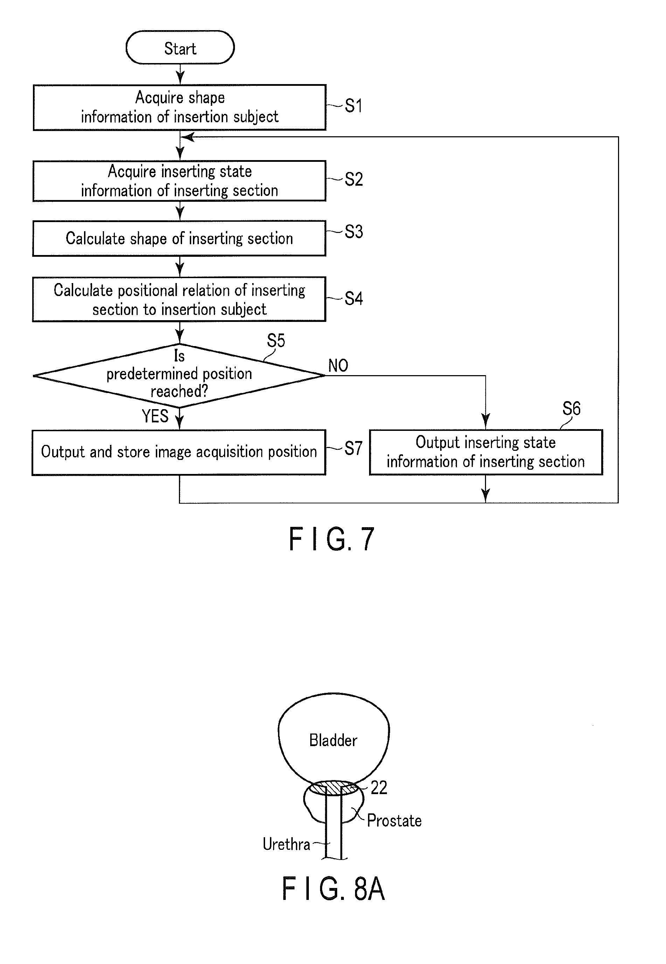

An operation of the insertion supporting device 6 of such a constitution as described above will be described with reference to FIG. 7. Here, for simplification of the description, there will be described, as an example, a case where the control section 66 controls the output state of the display output of the calculation result of the positional relation calculating section 63, i.e., the image acquisition position P (e.g., the intersection 72) in the output section 64, and controls the storage of the calculation result in the storage section 65 only at the start points of the display output and the storage.

First, the insertion subject shape acquiring section 62 acquires the insertion subject shape information (step S1). Afterward, the inserting state acquiring section 61 acquires the inserting state information of the inserting section 31 inserted into the insertion subject 2 (step S2). Furthermore, the positional relation calculating section 63 calculates a shape of the inserting section 31, on the basis of the position and direction of each of the bend detecting portions 51 of the inserting section 31 as the inserting state information (step S3). Afterward, on the basis of this calculated shape of the inserting section 31, the position and direction of the certain point of the inserting section 31 as the above acquired inserting state information and the above acquired insertion subject shape information, the positional relation calculating section calculates the positional relation of the inserting section 31 in relation to the insertion subject 2, i.e., the position of the inserting section distal end and the direction (axial direction) in which the distal end is directed (step S4).

Furthermore, the judging portion 67 of the control section 66 judges whether or not the inserting section distal end has reached the predetermined position (step S5). For example, taking a bladder as an example of the insertion subject 2, in the judgment of whether or not this inserting section distal end has reached the predetermined position, it is judged whether or not the distal end of the inserting section 31 inserted from an urethral opening that is the insertion port 21 has reached an inlet/outlet 22 of the bladder, i.e., a connecting portion between the bladder and a urethra as shown in FIG. 8A. Alternatively, the judgment can be performed by judging whether or not the inserting section distal end has reached a predetermined distance, e.g., a distance of about 2 cm from a bladder wall. In these judgments, it can be judged whether the inserting section distal end has reached the inlet/outlet 22 or the predetermined distance from the bladder wall, from the calculation results (the position of the inserting section distal end and the insertion subject shape information) output from the positional relation calculating section 63 or the above image acquired from the image acquisition section 32. Additionally, the judgments can be performed by judging, from the above image acquired from the image acquisition section 32, whether or not a blood vessel interval of the bladder wall has reached, e.g., about 100 pixels. In addition, also when the image acquisition section 32 is excessively close to a subject, the acquired image suitable for observation cannot be obtained, and hence this predetermined position can have a range. For example, in terms of the distance, the predetermined position can be in a range of about 2 cm mentioned above to about 5 mm from the bladder wall, and in terms of the pixels, the blood vessel interval of the bladder wall can be in a range of about 100 pixels mentioned above to about 500 pixels.

Here, in a case where the inserting section distal end has not reached the predetermined position yet, the control section 66 allows the output section 64 to display an inserting state of the inserting section 31 to the shape of the insertion subject 2, on the basis of part of the above calculation results output from the positional relation calculating section 63, i.e., the shape information of the inserting section 31, the position of the inserting section distal end and the direction (the axial direction) in which the distal end is directed, and the insertion subject shape information (step S6), and then the above operation is repeated from the above step S2.

In consequence, if the inserting section distal end reaches the predetermined position, the control section 66 allows the output section 64 to display and output the image acquisition position information indicating the image acquisition position P (e.g., the intersection 72) which is part of the above calculation results, and the control section also allows the storage section 65 to store the information (step S7). Afterward, the above operation is repeated from the above step S2.

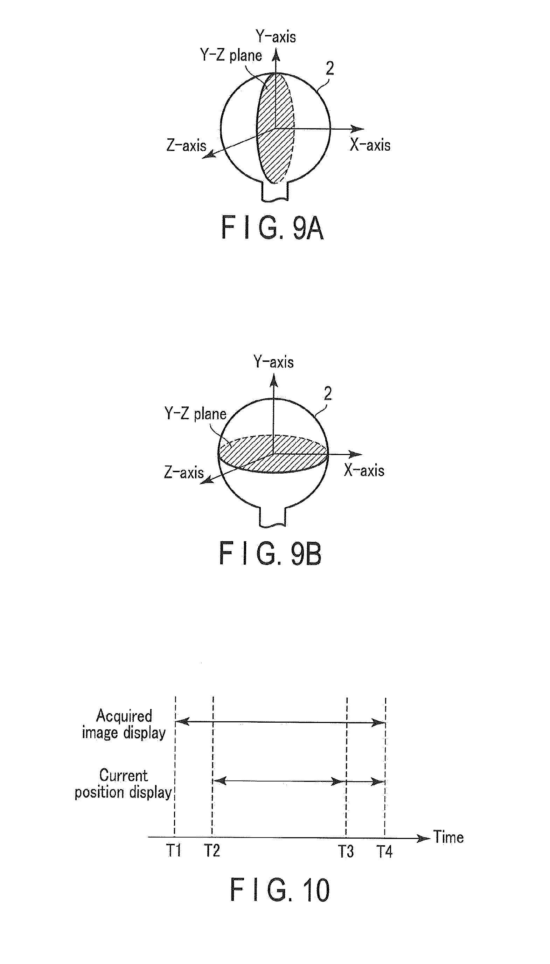

According to such an operation, the output section 64 performs such a display as shown in FIG. 1C. That is, in the present embodiment, the output section 64 displays and outputs the acquired image (an acquired image display 641) from the image acquisition section 32 and the inserting state (an inserting state display 642) of the inserting section 31. Here, the inserting state display 642 is displayed and output as two-dimensional views 6421 and 6422 that are the insertion subject shape information obtained by dividing the insertion subject 2 by a predetermined region. Furthermore, on the two-dimensional views 6421 and 6422 each obtained by dividing the insertion subject 2 by the predetermined region, information concerning the intersection 72 between the image acquisition position P, i.e., the insertion subject 2 and the axial direction (the image acquisition direction 71 of the inserting section distal end) is displayed and output. Here, the two-dimensional views 6421 and 6422 are produced by the control section 66 on the basis of the shape information of the inserting section 31. That is, the first two-dimensional view 6421 is a view showing a state where the shape of the insertion subject 2 is divided by a Y-Z plane and opened in a right-left direction in a coordinate of the insertion subject 2 as shown in FIG. 9A, and the second two-dimensional view 6422 is a view showing, as a view having a view point different from that of the first two-dimensional view 6421, a state where the shape of the insertion subject 2 is divided by an X-Z plane and opened in an upward-downward direction in the coordinate of the insertion subject 2 as shown in FIG. 9B. Furthermore, the control section 66 allows the output section 64 to display and output a current position display 643 as the information concerning the image acquisition position P on the two-dimensional views 6421 and 6422.

However, the current position display 643 is not always performed. That is, as shown in FIG. 10, when the insertion system starts the operation at a time T1, the acquired image display 641 is immediately started, but the current position display 643 on the two-dimensional views 6421 and 6422 is not displayed or output. Furthermore, for the first time at a point when the judging portion 67 of the control section 66 judges that the inserting section distal end has reached the predetermined position (a time T2), the display output of the current position display 643 on the two-dimensional views 6421 and 6422 is started. The display output of the acquired image display 641 and the display output of the current position display 643 on the two-dimensional views 6421 and 6422 are continued until the operation is ended by the insertion system at a time T4. Alternatively, as to the current position display 643 on the two-dimensional views 6421 and 6422, the judging portion 67 of the control section 66 judges that the inserting section distal end returns from the predetermined position, and at this time (a time T3), the display output may be ended.

It is to be noted that the current position display 643 may be the intersection 72 itself between the insertion subject 2 and the axial direction of the inserting section distal end, but as described above, when the display has the certain degree of range, e.g., the image acquisition region 73 or a region 74 of a part of the image acquisition region around the intersection 72, the display is more easily visually recognized.

Additionally, the output section 64 can display and output an inserting section shape schematic display 644 showing the shape of the inserting section 31, in addition to the current position display 643 as information concerning the current image acquisition position P. That is, the shape of the inserting section 31 and the position and direction of the inserting section distal end are calculated by the positional relation calculating section 63 as described above, so that the inserting state of the inserting section 31 inserted into the insertion subject 2 can be known, and the inserting section shape schematic display 644 can be performed.

Furthermore, the output section 64 can display and output a position locus display 645 by use of the calculation result stored in the storage section 65. This calculation result stored in the storage section 65 can be stored from the point (the time T2) when the inserting section distal end reached the predetermined position, on the basis of the above judgment of the judging portion 67. It is to be noted that the position locus display 645 is also allowed to have a certain degree of range in the same manner as in the current position display 643. Additionally, in this case, such display information as to achieve some identification display is preferably prepared by changing mutual colors, concentrations or patterns or by performing the position locus display 645 as a blinking display so that the current position display 643 and the position locus display 645 can be distinguished. A configuration of this identification display may be controllable by the control section 66 that receives an operator's selection by a switch operation. In this case, a switch can be disposed in the operating section 33 of the soft endoscope device that is the inserting tool 3, a foot switch, a region where operator's brain waves, muscular movement or the like is detectable, a case of the insertion supporting device 6, a treatment tool to be inserted into the inserting section 31 from the operating section 33 of the soft endoscope device, or the like.

In addition, the judging portion 67 may judge whether the inserting section distal end has reached the predetermined position, on the basis of the above calculation result from the positional relation calculating section 63 or the above acquired image from the image acquisition section 32 and/or in accordance with the above operation of the switch by the operator. That is, when the operator observes the acquired image displayed as the acquired image display 641 and determines that the inserting section distal end has reached the predetermined position, the operator operates the switch, whereby the judging portion 67 judges that the inserting section distal end has reached the predetermined position.

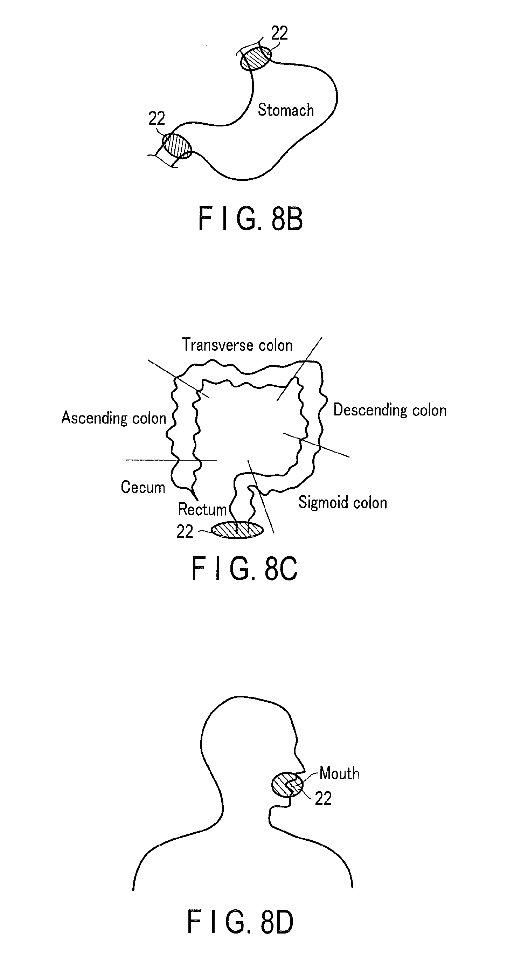

Furthermore, the operator may arbitrarily set the predetermined position by this operation of the switch. That is, the predetermined position can be changed in accordance with a type or a size of the insertion subject 2. For example, when the insertion subject 2 is the bladder, the inlet/outlet 22 is the connecting point between the urethra and the bladder as shown in FIG. 8A, but when the insertion subject 2 is a stomach, the inlet/outlet is a connecting portion of the stomach as shown in FIG. 8B, and when the insertion subject 2 is a large intestine in which a cecum, an ascending colon, a transverse colon, a descending colon, a sigmoid colon and a rectum are continuous, the inlet/outlet is an endpoint of the rectum as shown in FIG. 8C. Furthermore, when the insertion subject 2 is a human body, the inlet/outlet is a mouth as shown in FIG. 8D, and when the insertion subject is a piping line, the inlet/outlet is an opening or a branched region of the piping line.

In addition, there may be added a function that the operator can make a marking 646 in, for example, an already observed region or a region required to be observed hereafter or again (a region different from a peripheral image (which excludes the case of a known pattern), a characteristic pattern of a cancer, a blood outflow region, or the like) by the switch operation. For example, in accordance with the switch operation, the control section 66 controls the storage section 65 to store information of the corresponding region, and also controls the output section 64 to display the marking 646 in the region. In this case, as shown in FIG. 1C, the marking 646 is displayed on the two-dimensional views 6421 and 6422 showing the state where the shape of the insertion subject 2 is divided by the different planes in the coordinate of the insertion subject 2 and opened. Therefore, even a position hard to be recognized in one two-dimensional view (6421 in this example) can be displayed as a position easy to recognize in the other two-dimensional view (6422 in this example), and it is easy for the operator to identify a specific position of the insertion subject 2 at which the marking 646 is displayed.

This region of the marking 646 may be fixed to one of the intersection 72, the image acquisition region 73, and the region 74 of a part in the image acquisition region, or may arbitrarily be set by the operator. The marking 646 is enabled in this manner, thereby enabling confirmation of the observed region or the region required to be observed again in the insertion subject 2, a region that requires some treatment (removal, sampling, repair, or the like), or the like. Furthermore, it is possible to utilize the marking when the previous region required to be observed again is specified or the inserting section distal end is allowed to quickly reach the corresponding region in a case where the insertion subject 2 is observed again at a different opportunity. In addition, during the storage, when not only the information of the region provided with the marking 646 but also the inserting direction of the inserting section 31 and the like are stored, the marking can be utilized in a case where the confirmation is performed after the observation is performed, a case where the observation is next performed in the same state, or the like.

It is to be noted that when type information to specify each of the observed region, the region required to be observed again and the region required to be treated or symptom information/state information indicating the cancer, a polyp, a crack or the like can also be set and stored, a color or a shape of the display configuration is changed in accordance with the type information or the symptom information/state information, so that the operator can easily judge a meaning of each of the markings 646.

In addition, concerning the marking 646, the insertion supporting device 6 may be connected to a pointing device or a visual recognition device to allow the operator to designate any range or point on the acquired image display 641 or the two-dimensional view 6421 or 6422 displayed in the output section 64.

Additionally, in the two-dimensional views 6421 and 6422 shown in FIG. 1C, a region dividing display 647 is performed. In the views, there are displayed two or more regions which are divided in accordance with a common theory or stipulation of a learned society or for standard utilization, or divided by a predetermined dividing method. In this case, it becomes easy for the operator to identify certain positions in the insertion subject 2.

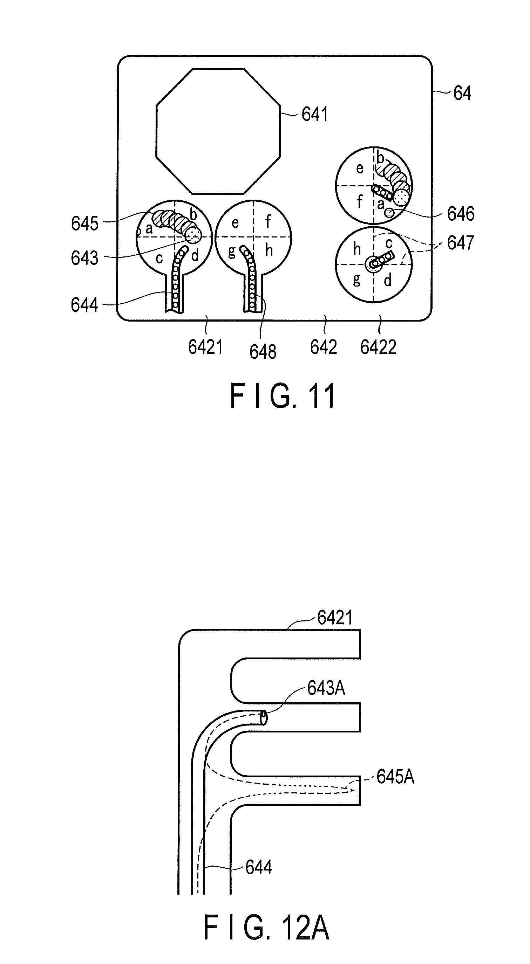

It is to be noted that as shown in FIG. 11, a bend detecting portion display 648 showing a position of the bend detecting portion 51 may be superimposed and displayed on the inserting section shape schematic display 644.

Here, the insertion and rotation detecting section 4 and the fiber shape sensor 5 optically detect the shape of the inserting section 31 in relation to the insertion subject 2 and a position and a direction of the image acquisition opening 34 as described above, but the detection may be performed by another method. For example, a coil is disposed in the vicinity of at least the image acquisition opening 34 in the inserting section 31 and a current is passed through the coil to generate a magnetic field which is received on the outside, or a magnetic field distribution generated on the outside is received by the coil, so that the position or direction of the coil, i.e., the image acquisition opening 34 can be detected. It is to be noted that when the coils are arranged in a longitudinal direction of the inserting section 31, the shape of the inserting section 31 can also be detected.

As described above, according to the present embodiment, the inserting state acquiring section 61 acquires the inserting state information (as regards insertion into the insertion subject 2) of (at least a part of) the inserting section 31 inserted into the insertion subject 2 (e.g., the position/direction of the certain point (the inserting section distal end) of the inserting section 31 and the position and direction of each of the bend detecting portions 51 of the inserting section 31. In addition, the insertion subject shape acquiring section 62 acquires the shape information of the insertion subject 2, and inputs these inserting state information and insertion subject shape information into the positional relation calculating section 63 to calculate the positional relation of the inserting section 31 in relation to the insertion subject 2 (the position or direction of the whole inserting section 31 or the inserting section distal end), and the output section 64 displays and outputs the calculation result of the positional relation calculating section 63, so that it is possible to supply information to judge which image of position of the insertion subject is being acquired. That is, when the inside of the insertion subject which cannot be seen directly by eye is observed with the soft endoscope device that is the inserting tool 3 having the inserting section 31, it is possible to understand a certain place of the insertion subject 2 which corresponds to the acquired image displayed in the output section 64 and a certain direction from which the place is being observed. In addition, it is possible to easily identify the observed region or an unobserved region of the insertion subject 2, which enables prevention of oversight.

Furthermore, in a case where the output section 64 displays and outputs the calculation result of the positional relation calculating section 63, i.e., the image acquisition position P, when the control section 66 is not present, the image acquisition position is always displayed and is output even if the output is not required, but in the present embodiment, the control section 66 executes control so that the display is performed only when necessary, so that useless calculation results are not displayed and a screen easy to understand (easy to see, hard to be mistaken, or which can quickly be judged) can be displayed. In addition, when the operator of the soft endoscope device that is the inserting tool 3 controls the control section 66, it is possible to securely display and output the only calculation results that are required for the operator (the current position, the observed position, an affected position, a position required to be extracted, etc.).

In addition, the insertion supporting device further includes the storage section 65 that stores at least part of the calculation results of the positional relation calculating section 63, and the storage section 65 stores an observation range of the insertion subject 2 displayed in the output section 64 (the positional relation of the inserting section 31 in relation to the insertion subject 2), so that it is possible to identify the observed region or a treated region of an inner wall of the insertion subject 2 with the inserting section distal end and the unobserved region or an untreated region. For example, when an observed or treated range displayed as the current position display 643 in the output section 64 is not erased but is left as the position locus display 645 during movement to another region, the observed or treated region and the unobserved or untreated region can easily be judged by the position locus display 645. According to this constitution, the whole insertion subject 2 can be observed or treated without oversight.

In addition, the insertion supporting device includes the image acquisition section 32 having the image acquisition opening 34 disposed in the inserting section 31, and displays the image acquired from the image acquisition section 32 in the output section 64, so that it is possible to directly acquire the image of the place of the insertion subject 2 observed with the inserting section distal end (the calculation result of the positional relation calculating section 63). Therefore, a state of an observation region can be obtained, and further, a change can specifically be detected by observing the same place (reaching the same place) again at a different time.

In addition, the control section 66 includes the judging portion 67 that judges, from the calculation result of the positional relation calculating section 63, whether the inserting section distal end has reached the predetermined position. Furthermore, when the judging portion 67 judges that the inserting section distal end has reached the predetermined position, the control section performs control of the output state of the output section 64, i.e., start/end of a continuous display output of the current position display 643 showing the image acquisition position P that is the calculation result of the positional relation calculating section 63, switching of display output contents/display output method (start/end, type, size or the like of the position locus display 645 or the marking 646), or the like. Here, the predetermined position is the inlet/outlet 22 (a position preset on shape information, e.g., an insertion opening of the insertion subject 2 or an inlet of the observation region) of the observation region of the insertion subject 2 (when the predetermined position is an organ, the organ is the predetermined organ), a known branched region, or a region where the acquired image includes a specific pattern (the cancer, the polyp, the crack or the like). It is to be noted that the size of the position locus display 645 or the marking 646 may be changed according to a distance between the inserting section distal end and the insertion subject 2, or may be set to an identifiable degree (a changeable size) in a case where the intersection 72 between the image acquisition direction 71 and the insertion subject 2 is displayed and output as the image acquisition position P. In addition, when the image acquisition section 32 is present, the region may be displayed and output in accordance with the size of the affected position or the crack in the acquired image.

Additionally, in a constitution where the control section 66 automatically executes the control of the output state of the output section 64 as described above when the inserting section distal end reaches the predetermined position of the insertion subject 2, the preset required region or state can only be displayed and output, and hence the operator of the inserting tool 3 can concentrate on the operation.

Here, the control section 66 controls the predetermined position in accordance with the switch operation of the operator, so that the required display output can be performed by an intention of the operator, and solely the operator's required information can be output securely. In addition, results judged by the operator (including a result hard to be automatically judged by image recognition such as pattern matching) can be distinguished and output. It is to be noted that there may be disposed one or more switches (for an instruction of the start/end of the current position display 643, for an instruction of the start/end of the position locus display 646, for an instruction of the marking 646, for a further separate marking instruction, and for a deletion instruction). When one switch is disposed, output states can be switched in accordance with the number of times or a pattern of on/off of the switch, successive function switching, or the like.

In addition, the control section 66 allows setting of a state of an observation (/operation) object region that is an insertion object region of the insertion subject 2, e.g., the cancer, the polyp or the crack and a state display range corresponding to a size or a breadth of the insertion object region in accordance with a setting operation of the operator, so that it is possible to set the output state or range on the basis of the operator's intention. Therefore, it is possible to arbitrarily set standards of re-observation or the like on the basis of standards of the operator or a field to which the operator belongs. That is, it is possible to selectively use the standards in accordance with respective fields such as a medical field and an industrial field. When the output state is automatically controlled, judgment standards of the control may be set beforehand.

In addition, the control section 66 allows the storage section 65 to store the acquired image, with the image being associated with the calculation result of the positional relation calculating section 63, so that as to the state of the predetermined region, a difference/change of the state can be obtained by comparing an observation state when the state was stored with a current observation state. That is, the acquired image of the observation range matches the calculation result of the positional relation calculating section 63, i.e., the image acquisition position P, so that it is possible to obtain information indicating a specific position of an image acquisition result, and it is also possible to accurately match changes of the same affected position identified at diagnosis.

In addition, the control section 66 allows the storage section 65 to store the acquired image, with the image being associated with the calculation result of at least the positional relation calculating section 63 and the symptom information, so that as to the state of the predetermined region, it is possible to obtain the difference/change between the observation state when the state was stored and the current observation state.

In addition, the control section 66 allows the output section 64 to display two or more different predetermined states by a distinguishable method; for example, to display markings that can be distinguished, as the calculation results of the positional relation calculating section 63, so that it is possible to apparently distinguish states (the current observation range, locus, affected position 1 (cancer, inflammation, defect, wound, corrosion, etc.), affected position 2 (cancer, inflammation, defect, wound, corrosion, etc.), ..., treated, progress required, etc.). When these states are stored in the storage section 65 beforehand, the region in the predetermined state can only be observed in a case where the same place is to be observed again at a different time, an observation efficiency can be improved, and further, a change condition of the region can be obtained.

It is to be noted that classification of the predetermined states can be selected by the operator of the inserting section 31. In this case, the predetermined states can be classified on the basis of the operator's intention, and hence a standard of re-observation or the like can arbitrarily be set by the operator or a standard of a field to which the operator belongs. That is, it is possible to use the device appropriately, in accordance with the field, such as a medical field and an industrial field.

In addition, the control section 66 executes control so that the output section 64 performs display in which region dividing displays are carried out in regions. In consequence, when the region dividing displays are carried out in a display screen in accordance with the common theory or stipulation of the learned society or so that the region for standard utilization can be seen, it is possible to easily understand which position is being observed in a case where the observation range is displayed.

It is to be noted that the control section 66 allows the output section 64 to display two or more different region dividing displays for the same region of the insertion subject 2, for example, to display the different two-dimensional views 6421 and 6422 so that the observation (/operation) object region and/or the positional relation of the inserting section 31 to the object region can be specified. In this case, when the three-dimensionally shown insertion subject 2 is two-dimensionally shown, it is possible to prevent a position corresponding to a depth direction from being unseen or being hard to be seen. Therefore, oversight regions or marking positions can securely be recognized (can easily be seen, can be hard to be mistaken, or can quickly be judged).

In addition, the control section 66 includes a function of an associated information calling portion that allows the output section 64 to display the information stored in the storage section 65, and hence it is possible to look back the observation result during the insertion even after the insertion, i.e., to look back the observed region and the unobserved region of the insertion subject 2, from a result of a viewing field (the image acquisition region 73) stored in the storage section 65 and output by the output section 64. Additionally, at the next insertion, the observation place of the previous time is stored, and hence the same region can easily be guided or specified. Furthermore, during the observation of the second time or after, when the previous information (the acquired image, symptom, size, etc.) of the same region is present, the information is called and displayed as associated information, so that on the spot, it is possible to judge the same region marked at the previous time, a difference from the previous time, or a progress condition.

In addition, when an insertion amount and a rotation amount of the inserting section 31 are detected by the insertion and rotation detecting section 4 and the shape of the inserting section 31 is detected by the fiber shape sensor 5, the insertion amount and rotation amount of the inserting section 31 in relation to the insertion subject 2 on the basis of a reference position such as the inlet/outlet 22 in relation to the insertion subject 2 and the shape of the inserting section 31 from the reference position can be detected, so that it is possible to detect the shape of the inserting section 31 in relation to the insertion subject 2 and the inserting state information as regards insertion into the insertion subject 2 (the position and direction of the image acquisition opening 34, or the like).

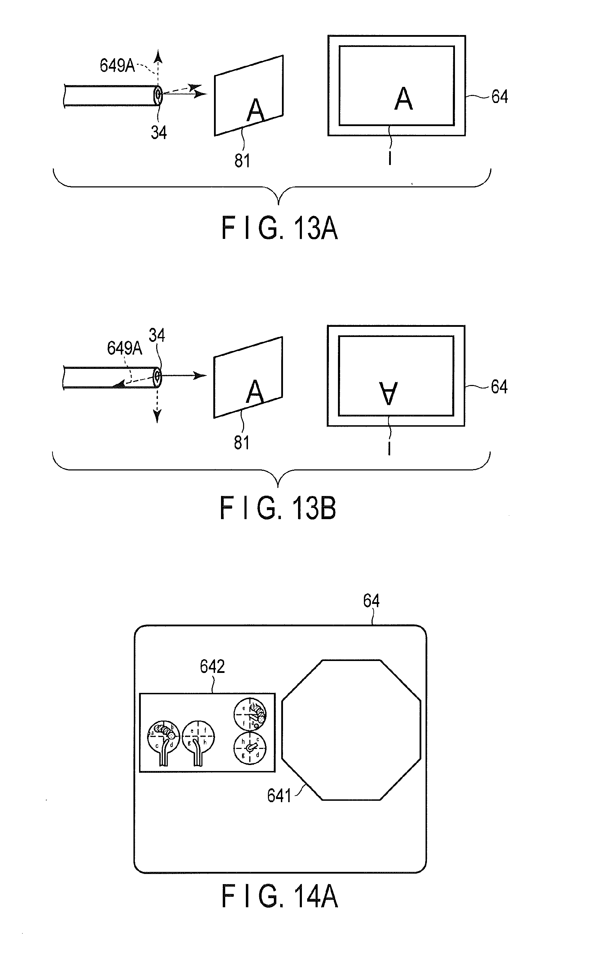

The description has been given as to the example where the image acquisition position is displayed. However, in addition to this position, there may be displayed the position and direction of the inserting section distal end and further, a history of the position and direction of the inserting section distal end. This will be described with reference to FIG. 12A to FIG. 12C and FIG. 13A and FIG. 13B. FIG. 12A to FIG. 12C show content displayed by the output section 64 when the inserting section 31 is inserted into the branched insertion subject 2.

The positional relation calculating section 63 calculates the position and direction of the inserting section distal end and outputs the same to the control section 66. It is to be noted that the calculation of the position and direction of the inserting section distal end is similar to the above calculation, and hence description thereof is omitted here.

FIG. 12A shows the inserting section shape schematic display 644 showing the shape of the inserting section 31 at the current time, a current position display 643A showing the current position of a distal position of the inserting section 31 (i.e., the position of the image acquisition opening 34), and a position locus display 645A showing a locus of the position of the image acquisition opening 34, on a two-dimensional view 6421A showing the shape of the insertion subject. It is to be noted that the position locus display 645 showing the locus of the image acquisition position is omitted. From the locus display of the distal position, it is possible to recognize a specific position of the insertion subject 2 through which the distal position (i.e., the position of the image acquisition opening 34) passes and a specific position of the insertion subject where the distal position is present at a current time.

When the distal position (i.e., the position of the image acquisition opening 34) is recognized, a specific position of the image acquisition object which is reached is recognized. When the current position is exactly recognized, the observation or treatment to be carried out at the current position or investigation of a path from the current position to a target position can be performed by using this information, without presuming that the current position would be this place. Therefore, it is not necessary to repeat trial and error in reaching the target position, nor is it necessary to confirm whether or not the target position was reached, by various methods including, for example, a method of observing the acquired image. As a result, there is a high possibility that the target position can be reached at one time by taking the path close to the shortest course from the current position to the target position, so that time can be reduced and furthermore, a situation concerning the position can be grasped, which leads to a calmed and assured operation.

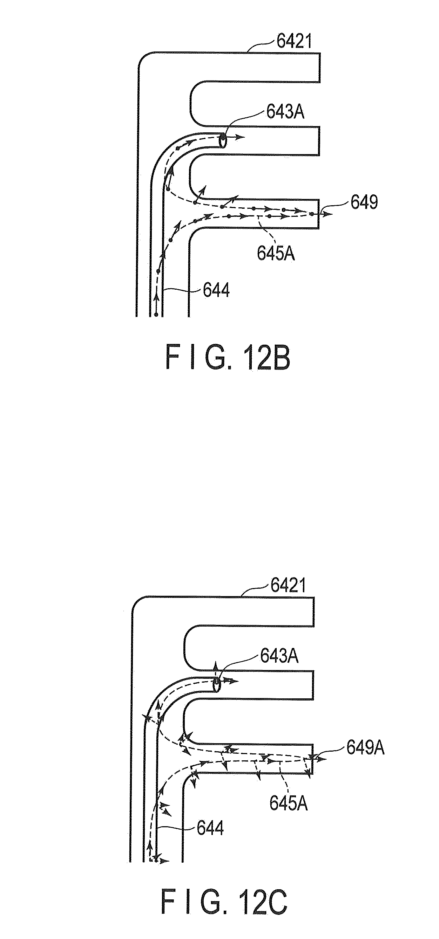

In addition to the position locus display 645A as the history of the distal position (i.e., the position of the image acquisition opening 34) shown in FIG. 12A, a direction in which the inserting section distal end (i.e., the image acquisition opening 34) is directed may be shown. FIG. 12B shows the direction in which the image acquisition opening 34 is directed, as the direction of the inserting section distal end by an arrow 649. In addition to the current position and direction of the image acquisition opening 34, information of directions at several positions on the locus of the image acquisition opening 34 is added by using the arrows 649. From the position locus display 645A of the distal position and the arrows 649 as displays of the directions, it is possible to recognize the position information of the image acquisition opening 34 at the inserting section distal end, i.e., the locus of the distal position, and a specific direction in which the image acquisition opening is directed while the position of the image acquisition opening changes.

It is to be noted that depending on the optical system for the image acquisition, in the present example, the direction in which the image acquisition opening 34 present at the inserting section distal end is directed is the center of the viewing field and is the middle of the acquired image.

When the distal position and direction are recognized, a position reached and a direction in the image acquisition object are recognized. An observation viewing field direction and the viewing field center are seen from the current position and direction. When the reaching position and direction or the observation viewing field direction and viewing field center are exactly recognized, it is possible to perform the observation or treatment to be carried out in accordance with the current position and direction, or the investigation of the path from the current position to the target position and the shape or operating method of the inserting section 31 during the movement, by use of this information without presuming that the current position and direction would be such the position and direction. In particular, when the direction of the inserting section distal end is recognized, it is possible to investigate an operating method or procedure such as insertion/extraction or bending for the purpose of reaching the target position or direction.

The direction of the inserting section distal end, i.e., the direction in which the image acquisition opening 34 is directed may three-dimensionally be shown to indicate the direction including a posture or rotation of the inserting section distal end.

When the rotation of a coordinate system fixed to the inserting section distal end, i.e., the coordinate system in which the position or posture of the inserting section distal end does not change is defined as "a three-dimensional direction" of the inserting section distal end, FIG. 12C shows the locus of the inserting section distal end in the three-dimensional direction. FIG. 12C shows the direction of the inserting section distal end, i.e., the direction in which the image acquisition opening 34 is directed, by use of arrows 649A of three directions (an x-direction, a y-direction, and a z-direction) to show the three-dimensional direction (the posture).

In addition, information concerning the three-dimensional direction including the rotation may be displayed together with the image acquired by the image acquisition section 32.

When the position and three-dimensional direction of the inserting section distal end are recognized in this manner, for example, the image acquisition direction including the rotation of the inserting section distal end at the image acquisition position is recognized. In addition, an influence of the rotation in the distal end direction can be taken into consideration during the treatment or the like other than the image acquisition.

As to the image acquisition direction including the rotation at the image acquisition position, for example, even in a case where the image acquisition opening 34 is directed in the same direction as shown in FIG. 13A and FIG. 13B, the image acquisition opening 34 to an image acquisition object 81 also rotates when the inserting section 31 rotates round the image acquisition direction. In FIG. 13A and FIG. 13B, the image acquisition opening rotates as much as 180°, and hence the upside and downside are reversed, and in this case, an acquired image I taken by the image acquisition section 32 is also displayed upside-down. It becomes possible to take this influence of the rotation in the image acquisition direction during the observation or treatment into consideration, thus the acquired image can exactly be grasped without mistaking the top and bottom of the image.

In addition, a supply configuration of the information is not limited to a display layout of the output section 64 shown in FIG. 1C.

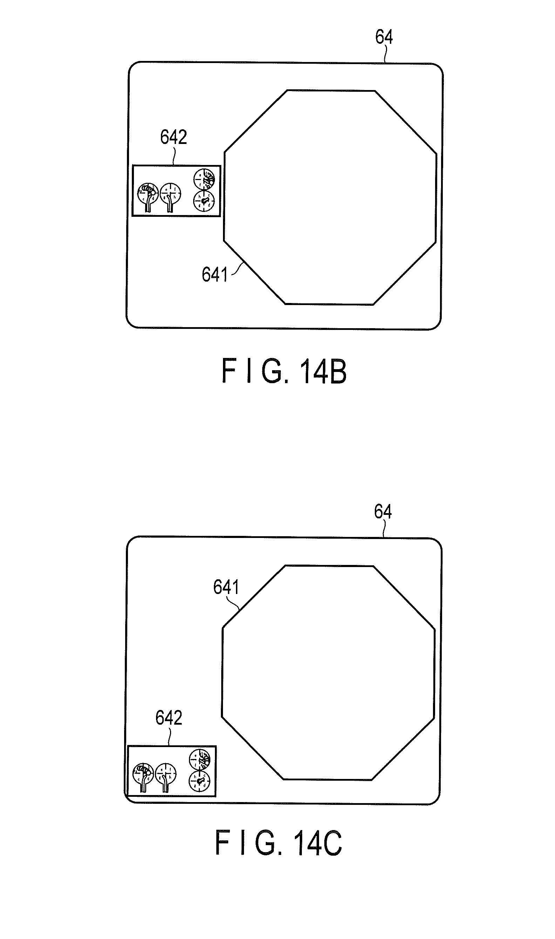

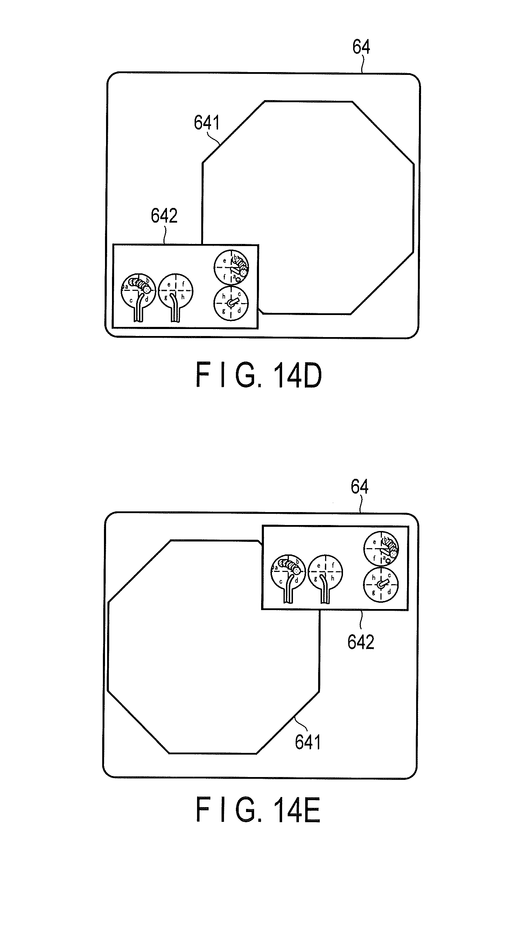

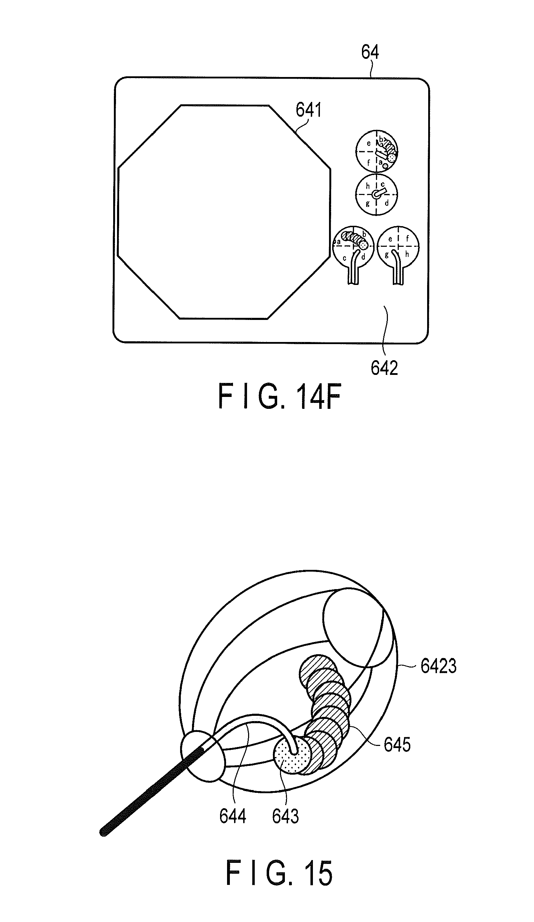

For example, as shown in FIG. 14A to FIG. 14F, the acquired image display 641 and the inserting state display 642 can be laid out at various sizes and positions and in various overlap conditions, respectively. In addition, from these display layouts, a layout that is felt to be easy to see by the operator may arbitrarily be selected and changed by the operator.

In addition, the inserting state display 642 is not limited to such a display as in the two-dimensional views 6421 and 6422. For example, a display may be carried out in a three-dimensional view 6423 as shown in FIG. 15.

### [Second Embodiment]

Next, a second embodiment of the present invention will be described.

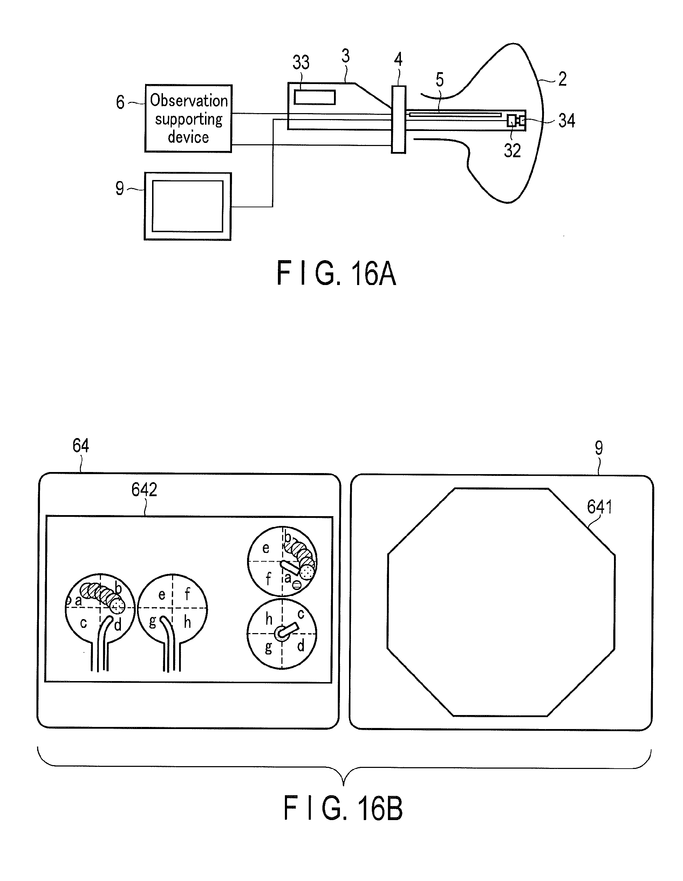

In the insertion system 1 concerned with the above first embodiment, a display section that displays an acquired image is constituted as the same device as the output section 64, but as shown in FIG. 16A, an insertion system 1 concerned with the present second embodiment further includes, as the display section, a display device 9 that is a device separate from the output section 64. In such a constitution, as shown in FIG. 16B, an inserting state display 642 is only carried out in the output section 64, and an acquired image display 641 is only carried out in the display device 9.

Therefore, when the output section 64 is disposed in the vicinity of the display device 9, a calculation result of a positional relation calculating section 63 and an image acquired by an image acquisition section 32 can substantially simultaneously be seen, and both of the result and the image can simultaneously be recognized, so that the calculation result of the positional relation calculating section 63 can be recognized without any burdens.

### [Third Embodiment]

Next, a third embodiment of the present invention will be described.

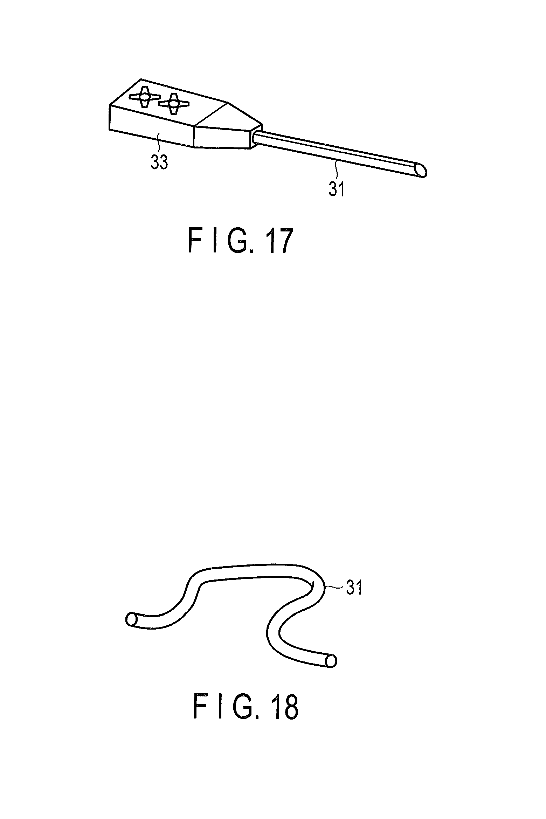

An insertion system 1 concerned with the present third embodiment is an example of a case where an inserting tool 3 is such a hard endoscope device as shown in FIG. 17. In this hard endoscope device, differently from the soft endoscope device in the above first embodiment (and the second embodiment), an inserting section 31 does not have flexibility, and hence a shape thereof is constant.

Therefore, it is not necessary to arrange coils in a longitudinal direction of a fiber shape sensor 5 or the inserting section 31, and it is also not necessary for a positional relation calculating section 63 to calculate shape information of the inserting section 31. The shape information of the inserting section 31 may only be stored or input in the positional relation calculating section 63 beforehand.

The other respects are similar to those of the insertion system concerned with the above first embodiment (and the second embodiment).

Also in the insertion system 1 concerned with this third embodiment, effects similar to those of the insertion system concerned with the first embodiment (and the second embodiment) can be obtained.

### [Fourth Embodiment]

Next, a fourth embodiment of the present invention will be described.

An insertion system 1 concerned with the present fourth embodiment is an example of a case where an inserting tool 3 is a treatment tool that does not have an image acquisition section such as a catheter as shown in FIG. 18. This treatment tool includes a flexible inserting section 31 similarly to the soft endoscope device in the above first embodiment (and the second embodiment).

Therefore, a constitution, an operation and a function/effect of a part other than a part concerning an acquired image are similar to those of the insertion system concerned with the above first embodiment (and the second embodiment).

The present invention has been described above on the basis of the embodiments, but needless to say, the present invention is not restricted to the abovementioned embodiments and various modifications or applications are possible within the gist of the present invention.

For example, a program of software to realize the function shown in the flowchart of FIG. 7 is supplied to a computer, and the computer executes this program to enable realization of the above function.

## Claims

1. An insertion system **characterized by** comprising:
an inserting section that is to be inserted into an insertion subject;
an inserting state acquiring section that acquires inserting state information of the inserting section;
an insertion subject shape acquiring section that acquires shape information of the insertion subject;
a positional relation calculating section that is input the inserting state information and the insertion subject shape information and calculates a positional relation of the inserting section to the insertion subject;
an output section that outputs the calculation result of the positional relation calculating section; and
a control section that controls an output state of the calculation result in the output section.

2. The insertion system according to claim 1, **characterized by** further comprising a storage section that stores at least a part of the calculation result of at least the positional relation calculating section.

3. The insertion system according to claim 1 or 2, **characterized by** further comprising:
an image acquisition section disposed in the inserting section; and
a display section that enables displaying an image acquired from at least the image acquisition section.

4. The insertion system according to any one of claims 1 to 3, **characterized in that** the control section includes a judging portion that judges, from the calculation result of the positional relation calculating section, whether an inserting section distal end has reached a predetermined position, and the control section controls an output state of the calculation result in the output section, when the judging portion judges that the inserting section distal end has reached the predetermined position.

5. The insertion system according to claim 4, **characterized in that** the predetermined position is one of an inlet/outlet of an observation region of the insertion subject, a known branched region, and a region where an acquired image includes a specific pattern.

6. The insertion system according to claim 4 or 5, **characterized in that** the control section automatically controls the output state of the output section when the inserting section distal end reaches the predetermined position of the insertion subject.

7. The insertion system according to claim 4 or 5, **characterized in that** the control section controls the predetermined position in accordance with a switch operation of an operator.

8. The insertion system according to any one of claims 1 to 7, **characterized in that** the control section enables setting of a state of an insertion object region of the insertion subject and a state display range corresponding to a size or a breadth of the insertion object region in accordance with a setting operation of an operator of the inserting section.

9. The insertion system according to claim 3, **characterized in that** the control section allows the storage section to store the image acquired from the image acquisition section, with the image being associated with the calculation result of the positional relation calculating section.

10. The insertion system according to claim 3, **characterized in that** the control section allows the storage section to store the image acquired from the image acquisition section, with the image being associated with the calculation result of at least the positional relation calculating section and symptom information.

11. The insertion system according to any one of claims 1 to 3, **characterized in that** the control section allows the output section to display the calculation result of the positional relation calculating section by two or more distinguishable methods.

12. The insertion system according to any one of claims 1 to 3, **characterized in that** the control section allows the output section to perform display in which region dividing displays are carried out in regions.

13. The insertion system according to any one of claims 1 to 3, **characterized in that** the control section allows the output section to perform display in which two or more different region dividing displays are carried out for the same region of the insertion subject.

14. The insertion system according to claim 2 or 3, **characterized in that** the control section includes an associated information calling portion that enables the output section to display information stored in the storage section.

15. The insertion system according to claim 3, **characterized in that** the output of the output section and the display of the display section are displayed in the same device or different devices placed in the vicinity of each other.

16. The insertion system according to any one of claims 1 to 3, **characterized by** further comprising a detecting section that detects an inserting state of the inserting section, and that
the detecting section includes at least one of:
a shape sensor that is mounted in the inserting section and detects a shape of the inserting section; and
an inserting section sensor that is disposed in an insertion port of the inserting section into the insertion subject and detects an insertion amount and a rotation amount when the inserting section passes through the inserting section sensor.

17. An insertion supporting device which supports insertion of an inserting section of an inserting tool into an insertion subject, the insertion supporting device
**characterized by** comprising:
an inserting state acquiring section that acquires inserting state information of the inserting section;
an insertion subject shape acquiring section that acquires shape information of the insertion subject;
a positional relation calculating section that is input the inserting state information and the insertion subject shape information and calculates a positional relation of the inserting section to the insertion subject;
an output section that outputs the calculation result of the positional relation calculating section as display information; and
a control section that controls an output state of the calculation result in the output section.

18. An insertion supporting method for use in an insertion supporting device which supports insertion of an inserting section of an inserting tool into an insertion subject, the method **characterized by** comprising:
an inserting state acquiring step of acquiring inserting state information of the inserting section;
an insertion subject shape acquiring step of acquiring shape information of the insertion subject;
a positional relation calculating step of being input the inserting state information and the insertion subject shape information, and calculating a positional relation of the inserting section to the insertion subject;
an output step of outputting the calculation result of the positional relation calculating step as display information; and
a control step that controlling an output state of the calculation result in the output step.

19. A program which allows a computer to execute:
an insertion state acquiring procedure of acquiring inserting state information of the inserting section in an insertion supporting device which supports insertion of the inserting section of an inserting tool into an insertion subject;
an insertion subject shape acquiring procedure of acquiring shape information of the insertion subject;
a positional relation calculating procedure of being input the inserting state information and the insertion subject shape information, and calculating a positional relation of the inserting section to the insertion subject;
an output procedure of outputting the calculation result of the positional relation calculating procedure as display information; and
a control procedure of controlling an output state of the calculation result in the output procedure.
